# EUROPÄISCHE PATENTSCHRIFT

(11) **EP 2 676 697 B1**
(45) Veröffentlichungstag und Bekanntmachung des Hinweises auf die Patenterteilung: **11.10.2017**
(21) Anmeldenummer: 13170599.8
(22) Anmeldetag: 05.06.2013
(51) Int. Cl.: A61N 1/39, A61B 5/042, A61N 1/05, A61N 1/08, A61N 1/368, A61N 1/37

(54) **Dislokationssensor**
Dislocation sensor
Capteur de dislocation

(30) Priorität: 20.06.2012 US 201261661813 P
(43) Veröffentlichungstag der Anmeldung: 25.12.2013
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- WO-A1-2012/128836
- US-A- 5 910 120
- US-A1- 2007 255 327
- US-A1- 2012 143 278
- US-B1- 6 347 249
- US-B2- 7 664 550

## Beschreibung

Die Erfindung betrifft ein implantierbares Herz-Therapie- und/oder Herz-Monitoring Gerät, beispielsweise einen Herzschrittmacher oder Kardioverter/Defibrillator, welches in der Lage ist, eine Dislokation einer Elektrodenleitung oder eines Elektrodenpols selbsttätig zu erkennen.

Implantierbare Herz-Therapie- und/oder Herz-Monitoring Geräte, z.B. Herzstimulatoren in Form von Herzschrittmachern oder Cardiovertern/Defibrillatoren, sind grundsätzlich bekannt. Solche Herzstimulatoren sind in der Regel an Elektrodenleitungen angeschlossen, die in einer Kammer eines Herzens oder in deren unmittelbarer Nähe Stimulations- und optional zusätzliche Defibrillationselektroden besitzen. Über eine Stimulationselektrode - genauer: einen oder mehrere Stimulationselektrodenpole - kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartig stimulierte Kontraktion einer Herzkammer wird im Rahmen dieser Beschreibung als stimuliertes Ereignis bezeichnet, ein Stimulationsimpuls, der eine ausreichende Intensität besitzt, eine stimulierte Kontraktion einer Herzkammer hervorzurufen, wird als "überschwellig" bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion, beispielsweise des rechten Atriums eines Herzens, wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Diese Zeit wird als Refraktärzeit bezeichnet. Die mit der Depolarisation und Repolarisation einhergehenden elektrischen Potenziale können wahrgenommen und deren zeitlicher Verlauf - als Elektrokardiogramm bezeichnet - ausgewertet werden.

In einem Elektrokardiogramm sind mit einer Kontraktion des Ventrikels einhergehende, eine Depolarisation der Herzmuskelzellen widerspiegelnde Aktionspotenziale als sogenannte Q-Zacke zu erkennen, während sich die mit der Entspannung des Myokards einhergehende Repolarisierung der Herzmuskelzellen in einer sogenannten T-Welle widerspiegelt.

Beim gesunden Menschen wird der jeweilige Herzrhythmus durch den vom autonomen Nervensystem gesteuerten Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlicher (intrinsischer) Ereignisse erfolgt durch Ableiten der elektrischen Potenziale des Myokards der jeweiligen Herzkammer mit Hilfe von Wahrnehmungselektroden, die Teil einer entsprechenden Elektrodenleitung sind. Dabei können die Wahrnehmungselektrodenpole gleichzeitig die Stimulationselektrodenpole sein und abwechselnd als Stimulations- und als Wahrnehmungselektrodenpol verwendet werden. Typischerweise ist für das Sensing - d.h. die Wahrnehmung intrinsischer Ereignisse - ein Wahrnehmungselektrodenpolpaar vorgesehen, das von zwei benachbarten Elektrodenpolen, nämlich einer Spitzenelektrode (Tip-Elektrode) und einer Ringelektrode, gebildet ist, von denen die Spitzenelektrode auch als Stimulationselektrodenpol dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Wahrnehmungselektrode aufweisen kann.

In Bezug auf die hierin verwendeten Bezeichnungen sei darauf hingewiesen, dass im Rahmen dieses Textes mit den Begriffen Stimulations- oder Wahrnehmungselektrode ein jeweiliger Elektrodenpol an einer Elektrodenleitung gemeint ist, also derjenige Teil einer Elektrodenleitung, über den Stimulationsimpulse abgegeben oder elektrische Potenziale aufgenommen werden. Es sei auch darauf hingewiesen, dass es auch üblich ist, mit "Stimulationselektrode" eine der Stimulation dienende Elektrodenleitung zu bezeichnen.

Die Wahrnehmungselektrodenpole sind im Betrieb des Herzstimulators mit entsprechenden Wahrnehmungseinheiten verbunden, die ausgebildet sind, ein jeweiliges über einen Wahrnehmungselektrodenpol (bzw. ein Wahrnehmungselektrodenpolpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Durch Dislokation ("Verrutschen" oder "Verschieben") einer Elektrodenleitung samt ihrer Wahrnehmungselektrodenpole kann es dazu kommen, dass sich Amplitude und /oder Form der über eine oder mehrerer Wahrnehmungselektrodenpole aufgenommen Signale ändert, ohne dass dies physiologisch bedingt ist. Dies stellt in Bezug auf eine zuverlässige Auswertung erfasster Signale ein Problem dar, so dass der Wunsch besteht, eine Dislokation einer Elektrodenleitung und/oder ihrer Elektrodenpole zu erkennen, zumal aus erfassten Signalen eine Reihe relevanter Therapieparameter abgeleitet werden.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkt der Abgabe eines jeweiligen Stimulationsimpulses in Abhängigkeit einer jeweiligen Stimulationsrate geplant, die dem physiologischen Bedarf eines Patenten entsprechen soll, also bei größerer Anstrengung beispielsweise höher ist. Hierzu kann ein Herzstimulator mit einem oder mehreren Aktivitätssensoren ausgestattet sein, der beispielsweise ein CLS-Sensor (CLS: Closed Loop Stimulation) sein kann.

In Bezug auf eine möglicherweise die Wahrnehmung von Ereignissen beinflussende Dislokation von Wahrnehmungselektrodenpolen sind Lösungsansätze bekannt, eine solche Dislokation zu erkennen. Bekannte Lösungsansätze zur Erkennung einer Dislokation einer Stimulationselektrode basieren auf der Auswertung der Wahrnehmungsamplitude, der E-lektrodenimpedanzen und der Stimulationsreizschwellen.

In US 7,664,550 ist ein Verfahren beschrieben, die Dislokation einer linksventrikulären Elektrodenleitung mit ihren Elektrodenpolen an einer veränderten Signalamplitude, Morphologie oder einem geänderten zeitlichen Abstand zum atrialen Signal zu erkennen.

Hiervon ausgehend liegt der Erfindung der Wunsch zugrunde, die Sensitivität und die Spezifizität der Dislokationserkennung möglichst zu verbessern.

Hierzu wird erfindungsgemäß ein Herz-Therapie- und/oder Herz-Monitoring Gerät vorgeschlagen, welches mit wenigstens einer Elektrodenleitung verbunden oder zu verbinden ist, die wenigstens einen ersten Wahrnehmungselektrodenpol aufweist, und welches wenigstens einen zweiten Wahrnehmungselektrodenpol aufweist oder mit einem solchen verbunden ist, wobei der erste und der zweite Wahrnehmungselektrodenpol im Betrieb des Herz-Therapie- und/oder Herz-Monitoring Gerätes relativ zueinander beweglich sind. Das Herz-Therapie- und/oder Herz-Monitoring Gerät weist eine Dislokations-Detektionseinheit auf, die sowohl mit dem ersten als auch mit dem zweiten Wahrnehmungselektrodenpol wenigstens mittelbar verbunden oder zu verbinden ist und die ausgebildet ist, zur Dislokations-Detektion einem jeweiligen kardialen Ereignis zuzuordnende Erfassungs- oder Wahrnehmungszeitpunkte an dem ersten Wahrnehmungselektrodenpol und an dem zweiten Wahrnehmungselektrodenpol relativ zueinander auszuwerten und ein Dislokations-Signal zu generieren, falls sich die relative zeitliche Beziehung von einem Typ kardialer Ereignisse zuzuordnenden Erfassungszeitpunkten über ein vorgegebenes oder jeweils bestimmtes Maß hinaus gegenüber einem zuvor erfassten Referenzwert ändert.

Mit einem derartigen Herz-Therapie- und/oder Herz-Monitoring Gerät lässt sich auch eine Dislokation einer linksventrikulären Coronar Sinus-Elektrode frühzeitig und zuverlässig erkennen, so dass beispielsweise mittels dieser Information eine CRT-Stimulation automatisch an infolge einer Dislokation geänderte Bedingungen angepasst werden kann.

Die Erfindung schließt nämlich die Erkenntnis ein, dass die bislang gebräuchlichen Verfahren zur Erkennung einer Elektrodendislokation nur eingeschränkt auf eine linksventrikuläre, also eine Coronar-Sinus-Elektrodenleitung anwendbar sind, da diese in einer Vene implantiert ist und bei einer Dislokation häufig "nur" einige Millimeter bis wenige Zentimeter innerhalb der Vene verschoben wird. Die Parameter Signalamplitude, Impedanz und Reizschwelle werden dabei unter Umständen. nur wenig beeinflusst, so dass eine solche Dislokation nicht zuverlässig erkannt werden kann.

Die genannten Nachteile der bislang gebräuchlichen Verfahren werden zwar mit der in US 7,664,550 vorgeschlagenen Methode des IEGM-Signalvergleiches grundsätzlich gelöst. Allerdings sind die in US 7,664,550 dargestellten Signalanalysemethoden nicht sensitiv genug, um normale IEGM-Exkusionen (unterschiedliche Herzfrequenzen, Extrasystolen) von einer tatsächlichen Elektrodendislokation sicher unterscheiden zu können. Ebenso ist es mit der in US 7,664,550 beschriebenen Vorrichtung nicht möglich, den Grad der Dislokation zu bestimmen und die CRT-Stimulation dementsprechend anzupassen.

Dokument WO 2012/128836 A1 offenbart ein Gerät nach dem Oberbegriff von Anspruch 1. Demgegenüber erlaubt es das erfindungsgemäße Herz-Therapie- und/oder Herz-Monitoring Gerät beispielweise, die Dislokation einer mehrpolaren Coronar Sinus-Elektrode zuverlässig zu erkennen, zu quantifizieren und die CRT-Stimulation dementsprechend automatisch anzupassen.
Vorzugsweise ist das Herz-Therapie- und/oder Herz-Monitoring Gerät ein implantierbarer Stimulator zur Stimulation des Herzens, der
- mit mindestens einer mehrpolaren Coronar Sinus-Elektrodenleitung (im Folgenden auch als CS-Elektrodenleitung bezeichnet) und
- mit mindestens einer weiteren, kardialen nicht CS-Elektrodenleitung verbunden ist,
- wobei die Elektrodenpole aller Elektrodenleitungen mit einer jeweiligen Wahrnehmungseinheit verbunden sind, die den Zeitpunkt der kardialen Erregung am jeweiligen Elektrodenpol erfasst.
Es ergibt sich eine Vorrichtung zur automatischen Erkennung der Dislokation einer mehrpolaren linksventrikulären Elektrode und eine Methode zur Bestimmung des Maßes der Dislokation, indem die Zeitpunkte der linksventrikulären Wahrnehmungen an mehreren Elektrodenpolen bezogen auf einen Referenzzeitpunkt erfasst, ausgewertet und die CRT-Stimulation automatisch an die geänderte Elektrodenposition angepasst wird. Vorzugsweise ist die Dislokations-Detektionseinheit ausgebildet, eine Zeitdifferenz zwischen zwei einem jeweiligen kardialen Ereignis zuzuordnenden Erfassungszeitpunkten als einem die relative zeitliche Beziehung beschreibenden Relativwert zu bestimmen, und diesen Relativwert mit einem Referenzwert zu vergleichen, der aus einem oder mehreren Relativwerten eines oder mehrerer jeweils vorangegangener Herzzyklen bestimmt ist. Verändert sich der Relativwert - also beispielsweise die Zeitdifferenz zwischen zwei Erfassungszeitpunkten - bedeutet dies, dass sich ein Erfassungszeitpunkt gegenüber dem am anderen Ort zum selben kardialen Ereignis bestimmten Erfassungszeitpunkt zeitlich verschoben hat. Eine derartige zeitliche Verschiebung ist ein Anzeichen für eine räumliche Verschie-bung des (ersten) Wahrnehmungselektrodenpols gegenüber dem zweiten Wahrnehmungselektrodenpol und damit auch ein Anzeichen für eine Dislokation der Elektrodenleitung.

Das Herz-Therapie- und/oder Herz-Monitoring Gerät weist vorzugsweise eine Stimulationseinheit auf, die mit einem Stimulationselektrodenpol verbunden oder zu verbinden ist, wobei die Dislokations-Detektionseinheit dazu ausgebildet ist, die relative zeitliche Beziehung der Erfassungszeitpunkte eines jeweils stimulierten Ereignisses zu bestimmen und für eine Dislokations-Detektion heranzuziehen. Indem als Ereignis ein stimuliertes Ereignis für eine Dislokations-Detektion generiert wird, ergibt sich eine definierte kardiale Konstellation, bei der der Stimulationsort bekannt und reproduzierbar ist, was die Spezifizität der Dislokations-Detektion verbessert.

Hierbei ist die Stimulationseinheit vorzugsweise eine atriale Stimulationseinheit und der Stimulationselektrodenpol ist vorzugsweise ein atrialer Stimulationselektrodenpol zur Platzierung im Atrium eines Herzens.

In einem besonders bevorzugten Fall ist die (erste) Elektrodenleitung mit dem ersten Wahrnehmungselektrodenpol eine Coronar-Sinus-Elektrodenleitung. Bei einer derartigen Ausführungsvariante kommen die Vorteile der Erfindung besonders zum Tragen, weil bisher bekannte Ansätze zur Dislokationserkennung im Falle von CS-Elektrodenleitungen nicht zuverlässig genug sind.

Der zweite Wahrnehmungselektrodenpol ist vorzugsweise Teil einer rechtsventrikulären oder rechtsatrialen Elektrodenleitung.

Die Dislokations-Detektionseinheit umfasst vorzugsweise eine Auswerteeinheit, die im Rahmen einer Zeitanalyse jeweilige Zeitdifferenzen (von jeweiligen Erfassungs- oder Wahrnehmungszeitpunkten) der Erregung bezogen auf ein von der nicht CS-Elektrodenleitung stammendes Referenzsignal derart auswertet, dass eine Dislokation bei hinreichender zeitlicher Verschiebung der Wahrnehmungszeitpunkte angezeigt wird und der Grad der Verschiebung durch Auswertung von mindestens zwei CS-Elektrodenpolen bestimmt wird.

Vorzugsweise umfasst das Herz-Therapie- und/oder Herz-Monitoring Gerät eine Stimulationssteuereinheit die mit wenigstens einer Stimulationseinheit verbunden und ausgebildet ist, die Abgabe eines jeweiligen Stimulationsimpulses über wenigstens einen bestimmbaren Stimulationselektrodenpol in Abhängigkeit des Dislokationssignals zu steuern.

In diesem Zusammenhang ist es besonders bevorzugt, wenn die Dislokations-Detektionseinheit ein Dislokationssignal liefert, das einen Grad einer zeitlichen Verschiebung von Erfassungs- oder Wahrnehmungszeitpunkten gegenüber dem zuvor erfassten Referenzwert widerspiegelt - also den Grad der Veränderung der Zeitdifferenz zwischen dem Erfassungszeitpunkt an dem ersten Wahrnehmungselektrodenpol und an dem zweiten Wahrnehmungselektrodenpol. Dann kann die Stimulationssteuereinheit in einer besonders bevorzugten Ausführungsvariante einen von mehreren Stimulationselektrodenpolen in Abhängigkeit des Grades der zeitlichen Verschiebung bestimmen.

Falls die (erste) Elektrodenleitung mit dem (wenigstens einen) ersten Wahrnehmungselektrodenpol eine CS-Elektrodenleitung ist, ist die Stimulationssteuereinheit vorzugsweise dazu ausgebildet eine automatische Umschaltung des CS-Stimulationsortes in Abhängigkeit des Grades der Dislokation oder aber eine Abschaltung der Stimulation bei höhergradiger Dislokation zu bewirken.

Vorzugsweise ist die Dislokations-Detektionseinheit dazu ausgebildet, eine Zeitanalyse zum Detektieren einer Dislokation grundsätzlich nur bei einer durch das Herz-Therapie- und/oder Herz-Monitoring Gerät erkannten oder eingestellten, definierten kardialen Konstellation durchzuführen, um so eine hinreichende Spezifität und Sensitivität erreichen zu können.

Eine solche definierte kardiale Konstellation kann dabei durch eine Überstimulation des Atriums (in einem AAI oder A00 Stimulationsmodus) etwas oberhalb der Eigenfrequenz eingestellt werden.

Alternativ kann eine solche definierte kardiale Konstellation durch eine Überstimulation des rechten Ventrikels (in einem RV-VVI oder RV-V00 Stimulationsmodus) etwas oberhalb der Eigenfrequenz eingestellt werden. Letzteres bietet sich für Patienten mit abladiertem AV-Knoten an.

Auch kann die Dislokations-Detektionseinheit ausgebildet sein, dass sie eine solche definierte kardiale Konstellation als gegeben erkennt, wenn das Herz-Therapie- und/oder Herz-Monitoring Gerät eine als körperliche Ruhe zu klassifizierende Phase erkennt. Letzteres kann z.B. der Fall sein, wenn ein Aktivitätssensor, z.B. ein Akzelerometer ein Ausgangssignal liefert, das auf körperliche Ruhe hinweist und/oder wenn ein Zeitgeber ein Zeitsignal liefert, das eine typische Ruhezeit (z.B. nächtliche Schlafenszeit) anzeigt.

Die Dislokations-Detektionseinheit kann auch ausgebildet sein, eine Dislokations-Detektion retrospektiv durchzuführen, indem sie Erfassungszeitpunkte heranzieht, die bereits in zurückliegender Zeit aufgenommen wurden. Vorzugsweise sind dies Erfassungszeitpunkte, die in einem Zeitraum aufgenommen wurden, an dem eine jeweils (z. B. tageweise) minimale Herzfrequenz vorlag.

Alternativ oder zusätzlich kann die Dislokations-Detektionseinheit ausgebildet sein, dass sie eine solche definierte kardiale Konstellation als gegeben erkennt, wenn das Herz-Therapie- und/oder Herz-Monitoring Gerät eine Phase niedriger Herzfrequenzvariabilität erfasst. Hierzu kann das Herz-Therapie- und/oder Herz-Monitoring Gerät ausgebildet sein, den sogenannten SDANN-Wert (Standard Deviation of the Averages of NN (Normal Sinus to Normal Sinus)) zu bestimmen. Der SDANN Wert ist die Standardabweichung der NN-Intervalle (der R-R Intervalle) vom Mittelwert in einer begrenzten Zeitperiode von 5 Minuten.

Alternativ oder zusätzlich kann die Dislokations-Detektionseinheit ausgebildet sein, eine Dislokations-Detektion nur vorzunehmen, wenn sich die Herzfrequenz in einem definierten Herzfrequenzfenster befindet, um so z.B. auch Patienten mit frequenzabhängigen Schenkelblöcken erfassen zu können. Ein derartiges definiertes Herzfrequenzfenster stellt somit ebenfalls eine im Sinne dieser Beschreibung definierte kardiale Konstellation dar.

Eine definierte kardiale Konstellation, auf die die Dislokations-Detektionseinheit anspricht, kann auch mehrere der vorgenannten Aspekte beinhalten, d.h. sich durch eine Kombination der vorgenannten Bedingungen auszeichnen.
In dem Fall, in dem die (erste) Elektrodenleitung mit dem (wenigstens einen) ersten Wahrnehmungselektrodenpol eine CS-Elektrodenleitung ist, ist die Dislokations-Detektionseinheit vorzugweise ausgebildet, eine Zeitanalyse zur Dislokations-Detektion durchzuführen, die eine Zeit-Korrektur basierend auf einer interventrikulären Signallaufzeit beinhaltet. Die interventrikuläre Signallaufzeit wird hierzu vorzugsweise an mindestens 2 CS-Elektrodenpolen gemessen. Auf diese Weise kann die Spezifität der Dislokations-Detektion erhöht werden.
Ein derartiges Herz-Therapie- und/oder Herz-Monitoring Gerät erlaubt in seiner bevorzugten Ausführungsform eine zuverlässige Dislokations-Detektion auch im Falle einer angeschlossenen CS-Elektrodenleitung sowie zusätzlich eine Quantifizierung der Dislokation und basierend darauf eine Anpassung des (linksventrikulären) Stimulationsortes.
In seiner bevorzugten Ausführungsform ist das Herz-Therapie- und/oder Herz-Monitoring Gerät ein implantierbarer biventrikulärer Herzschrittmacher und/oder Kardioverter/Defibrillator. Mit einem solchen Gerät kann insbesondere eine kardiale Resynchronisations-Therapie (CRT) durchgeführt werden.

Die Erfindung ist in Anspruch 1 definiert. Vorteilhafte Ausführungsbeispiele sind in den abhängigen Ansprüchen definiert.

Die Erfindung soll nun anhand von Ausführungsbeispielen mit Bezug auf die Figuren näher erläutert werden. Die Figuren zeigen in
- Fig. 1: Ein System mit einem Herz-Therapie- und/oder Herz-Monitoring Gerät als implantierbarer CRT-Stimulator in Form eines Dreikammer-ICD-Systems;
- Fig. 2: die Hauptbestandteile des Herz-Therapie- und/oder Herz-Monitoring Gerätes aus Figur 1;

- Fig. 3: die Ausgangsposition des Elektrodensystems und die zeitlichen Bezüge der linksventrikulären Wahrnehmungen gegenüber den als Referenzzeiten möglichen Signalen;
- Fig. 4: eine moderat dislozierte CS-Elektrode (LV);
- Fig. 5: ein Blockschaltbild einer Dislokations-Detektionseinheit;
- Fig. 6: ein Blockschaltbild einer alternativen Ausführungsvariante; und
- Fig. 7: einen Ablaufplan einer erfindungsgemäßen Implementierung der Dislokationsanalyse.

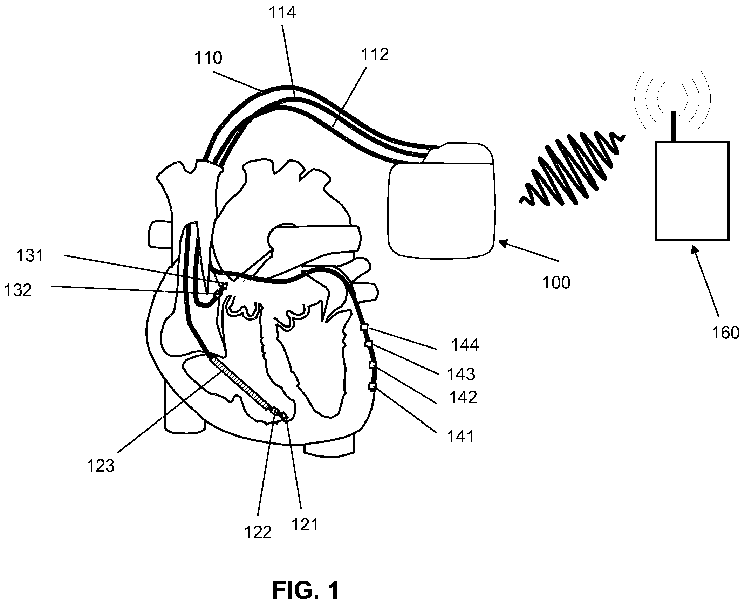

In Figur 1 ist als Beispiel für ein Herz-Therapie- und/oder Herz-Monitoring Gerät ein Dreikammer-ICD-System dargestellt. Diese umfasst eine implantierbares Gerät 100 (als Herz-Therapie- und/oder Herz-Monitoring Gerät), der mit mehreren implantierbaren Elektrodenleitungen 110, 112 und 114 verbunden ist. Zur rechtsventrikulären Wahrnehmung und Stimulation ist eine rechtsventrikuläre (RV-) Elektrodenleitung 110 vorgesehen, die eine rechtsventrikuläre Tipelektrode (RV Tip) 121 und eine rechtsventrikuläre Ringelektrode (RV Ring) 122 am distalen Ende umfasst. Über die RV-Tipelektrode 121 können im Betrieb bei Bedarf rechtsventrikuläre Stimulationsimpulse für eine biventrikuläre CRT-Stimulation abgegeben werden. Zur Defibrillationsschockabgabe sind an dieser Elektrodenleitung 110 eine distale Schockwendel (RV Coil) 123 und optional auch eine proximale Schockwendel (nicht dargestellt) angebracht. Die Gegenelektrode bildet hier das zumindest teilweise leitfähige Gehäuse des implantierbaren Gerätes 100.

Eine rechtsatriale Elektrodenleitung 112 weist an ihrem distalen Ende einen bipolare Wahrnehmungs- und Stimulationspol mit einer rechtsatrialen Tipelektrode (RA Tip) 131 und einer rechtsatrialen Ringelektrode (RA Ring) 132 auf und dient der Wahrnehmung des atrialen Rhythmus und bei Bedarf der atrialen Stimulation.

Außerdem umfasst das System auch eine linksventrikuläre CS-Elektrodenleitung zur Abgabe von linksventrikulären Stimulationsimpulsen zur CRT über einen oder mehrere von insgesamt vier linksventrikulären (CS-) Stimulationselektrodenpolen 141, 142, 143 und 144. Der linksventrikuläre Stimulationselektrodenpol 141 wird auch als linksventrikuläre Tipelektrode (LV Tip) bezeichnet. Die linksventrikulären Elektrodenpole 142, 143 und 144 werden auch als linksventrikuläre Ringelektroden (LV Ring) bezeichnet.

Für eine Kommunikation mit externen Programmier- und Steuer- und Datenübertragungsgeräten 160 ist in dem implantierbaren Gerät 100 eine drahtlose, bidirektionale Telemetrieeinheit vorgesehen.

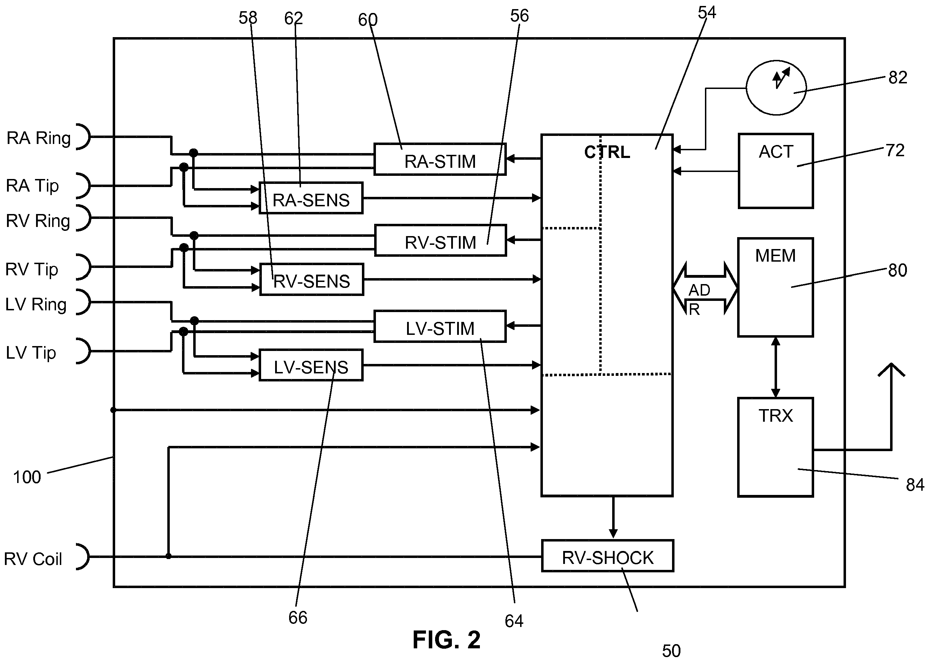

Figur 2 zeigt die Hauptbestandteile des implantierbaren Gerätes 100 aus Figur 1 in der Ausführungsform eines implantierbaren Herzstimulators. Auf der linken Seite sind die elektrischen Anschlüsse für die verschiedenen Elektroden 121 (RA Tip), 122 (RA Ring), 131 (RV Tip) und 132 (RV Ring)dargestellt. Die Schockelektroden 123 sind über den Anschluss RV Coil mit einem rechtsventrikulären Schockimpulsgenerator 50 verbunden. Der Schockgenerator 50 ist mit einer Stimulations-steuereinheit 54 verbunden, die den Schockimpulsgenerator 50 bei Bedarf zur Erzeugung und Abgabe eines Defibrillationsschocks ansteuert.

Der Anschluss für die rechtsventrikuläre Tipelektrode 131 (RV Tip) sowie der Anschluss für die rechtsventrikuläre Ringelektrode 132 (RV Ring) sind jeweils sowohl mit einer rechtsventrikulären Stimulationseinheit 56 als auch mit einer rechtsventrikulären Wahrnehmungseinheit 58 verbunden. Sowohl die rechtsventrikuläre Stimulationseinheit 56 als auch die rechtsventrikuläre Wahrnehmungseinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss für die rechtsventrikuläre Ringelektrode RV Ring 132 die rechtsventrikuläre Spitzenelektrode RV Tip 131 abzugeben. Alternativ ist es auch möglich, dass das Gehäuse des implantierbaren Gerätes 100 eine neutrale Elektrode bildet und die rechtsventrikuläre Stimulationseinheit 56 mit dem Anschluss für die rechtsventrikuläre Ringelektrode RV Tip 131 und dem Gehäuse als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einmal das vollständige Herzgewebe (Myokard) einer Herzkammer erregt, sondern nur die Herzmuskelzellen in unmittelbarer Umgebung der rechtsventrikulären Spitzenelektrode RV Tip 131. Diese Erregung breitet sich dann durch natürliche Reizleitung über den gesamten rechten Ventrikel weiter aus und sorgt so für eine stimulierte Kontraktion des rechten Ventrikels.

Die rechtsventrikuläre Wahrnehmungseinheit 58 ist dazu ausgebildet, an dem Anschluss für die rechtsventrikuläre Ringelektrode RV Ring 132 und die rechtsventrikuläre Tippelektrode RV Tip 131 anliegende elektrische Potenziale zunächst durch einen Eingangsverstärker zu verstärken und zu filtern. Weiterhin ist die rechtsventrikuläre Wahrnehmungseinheit 58 ausgebildet, den Verlauf der an ihren Eingängen anliegenden elektrischen Signale derart auszuwerten, dass die rechtsventrikuläre Wahrnehmungseinheit 58 selbsttätig eine natürliche (intrinsische), d.h. selbsttätige, Kontraktion des rechten Ventrikels detektiert. Dies kann beispielsweise dadurch geschehen, dass der Verlauf des an den Eingängen der rechtsventrikulären Wahrnehmungseinheit 58 anliegenden Signals mit einem Schwellenwert verglichen wird. Typischerweise ist die größte Amplitude des Signals in Form der sogenannten R-Zacke kennzeichnend für eine natürliche Kontraktion des rechten Ventrikels, die durch Schwellenwertvergleich detektiert werden kann. Die rechtsventrikuläre Wahrnehmungseinheit 58 gibt daraufhin ein entsprechendes, eine natürliche Kontraktion des rechten Ventrikels anzeigendes Ausgangssignal an die Stimulationssteuereinheit 54 aus. Der Zeitpunkt, an dem der Schwellenwert überschritten wird, ist der Erfassungszeitpunkt für das jeweilige Ereignis.

In gleicher Art und Weise können auch für die Schockelektroden eine oder mehrere (in Fig. 2 nicht dargestelle) Wahrnehmungseinheiten vorgesehen sein. Diese Wahrnehmungseinheit oder Wahrnehmungseinheiten sind bevorzugt ausgebildet, Signale zwischen den Schockelektroden, zwischen der Schockelektrode 123 und dem Gehäuse des implantierbaren Gerätes 100 oder zwischen der anderen Schockelektrode und dem Gehäuse des implantierbaren Gerätes 100 zu erfassen.

In analoger Weise sind der Anschluss für die rechtsatriale Spitzenelektrode 121 (RA Tip) und der Anschluss für die rechtsatriale Ringelektrode 122 (RA Ring) sowohl mit einer rechtsatrialen Stimulationseinheit 60 als auch mit einer rechtsatrialen Wahrnehmungseinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechts-ventrikulären Stimulationsimpulse. Die rechtsatriale Wahrnehmungseinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Wahrnehmungseinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Spitzenelektrode 141 LV Tip und die Anschlüsse für die linksventrikulären Ringelektroden LV Ring 142, 143 und 144 (aus Vereinfachungsgründen ist nur ein Anschluss LV Ring dargestellt) mit einer links-ventrikulären Stimulationseinheit 64 und einer linksventrikulären Wahrnehmungseinheit 66 verbunden. Die linksventrikuläre Stimulationseinheit 64 und die linksventrikuläre Wahrnehmungseinheit 66 sind ebenso mit der Stimulationssteuereinheit 54 verbunden. Beide funktionieren ähnlich wie die bereits beschriebenen Stimulationseinheiten 56 und 60 und Wahrnehmungs-Einheiten 58 und 62. Nicht in Figur 2 dargestellt ist eine Umschalteinheit, die es ermöglicht, beliebige Kombinationen der Elektroden 141, 142, 143 und 144 mit den Eingängen der linksventrikulären Stimulationseinheit 64 und die linksventrikulären Sensing-Einheit 66 zu verbinden. Um die gleichzeitige Erfassung mehrerer mit linksventrikulärer Signale zu ermöglichen, kann für jede der linksventrikulären Elektroden 141, 142, 143, 144 eine eigene, in Figur 2 nicht dargestellte, Sensing-Einheit vorgesehen sein. Analog kann auch für die gleichzeitige Abgabe mehrerer linksventrikulärer Stimulationsimpulse über mehrere der linksventrikulären Ringelektroden 141, 142, 143, 144 eine eigene, in Figur 2 nicht dargestellte, Stimulationseinheit vorgesehen sein.

Als weiterer Bestandteil des implantierbaren Gerätes 100 ist ein Beschleunigungssensor 72 mit der Stimulationssteuereinheit 54 verbunden und in das Gehäuse des implantierbaren Gerätes 100 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst. Das Akzelerometer-Ausgangssignal kann auch zum Bestimmen von Ruhephasen genutzt werden, in denen bevorzugt eine Dislokationsdetektion stattfinden kann.

Weiterhin umfasst das implantierbare Gerät 100 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 160 zu übertragen oder Programmierbefehle seitens des externen Geräts 160 zu dem implantierbaren Gerät 100 zu übertragen und in der Speichereinheit 80 zu speichern.

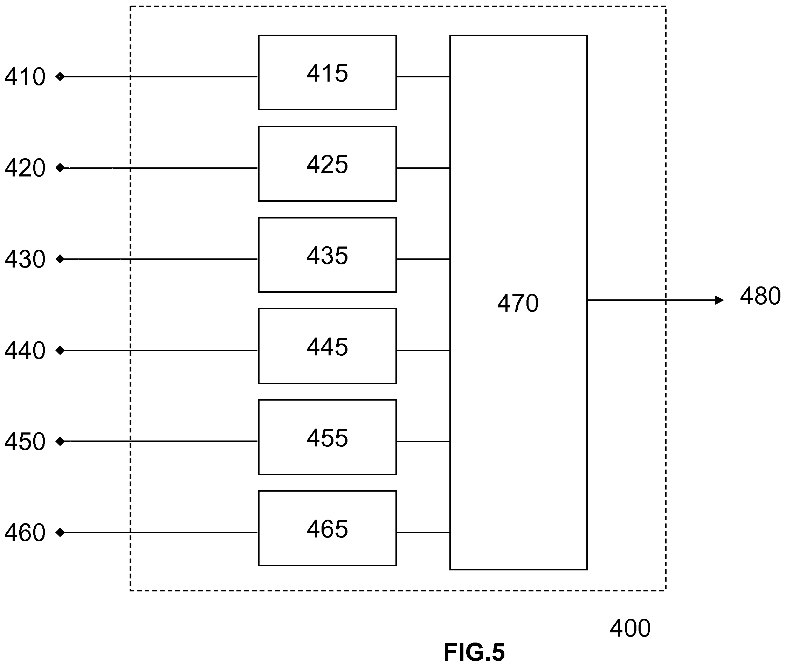

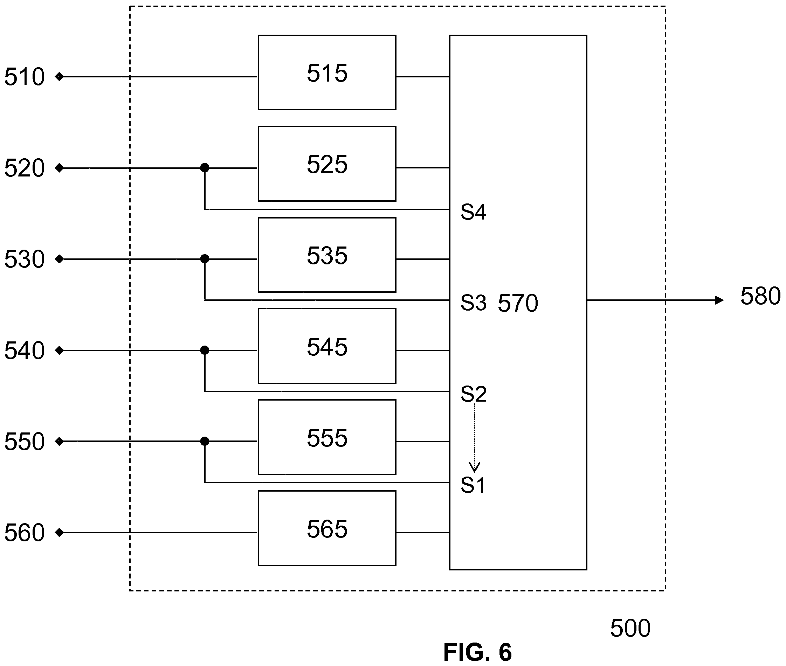

Die eingangs beschriebene Dislokations-Detektionseinheit und deren Auswerteeinheit sind in Figur 2 nicht dargestellte Bestandteil der Stimulationssteuereinheit 54 und in Figuren 5 und 6 dargestellt.

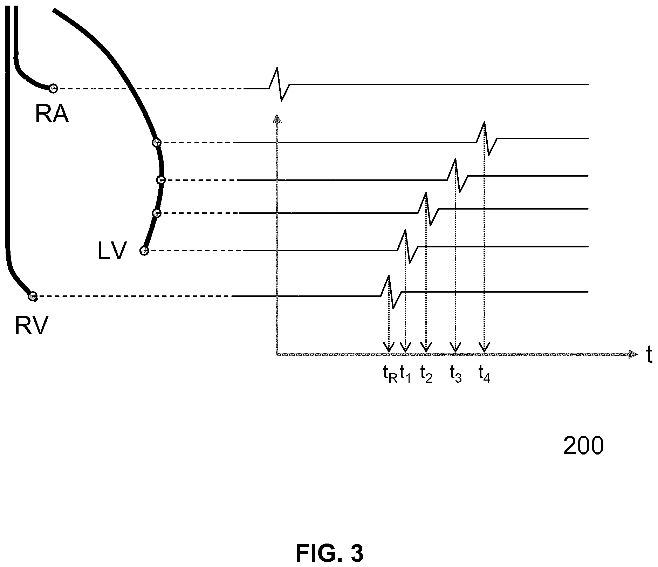

In Figur 3 sind die Ausgangsposition des Elektrodensystems und die zeitlichen Bezüge der linksventrikulären Wahrnehmungen (t1...t4) gegenüber den als Referenzzeiten möglichen Signalen im rechten Atrium (RA=0) oder rechten Ventrikel (RV=tR) dargestellt. Die linksventrikulären Wahrnehmungen erfolgen über die vier linksventrikulären (CS-) Stimulationselektrodenpolen 141, 142, 143 und 144 die in diesem Sinne jeweils als erste Wahrnehmungselektrodenpole wirken. Der Wahrnehmungselektrodenpol an der rechtsatrialen Elektrodenleitung 112 oder der Wahrnehmungselektrodenpol an der rechtsventrikulären Elektrodenleitung 110 bildet jeweils den zweiten (Referenz-) Wahrnehmungselektrodenpol.

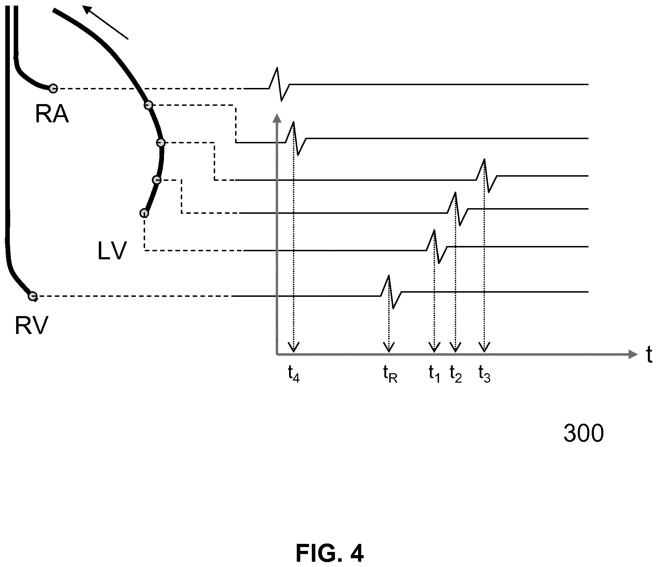

In Figur 4 ist eine dislozierte CS-Elektrodenleitung (LV) dargestellt. Diese hat sich gegenüber Figur 2 nun um einige Millimeter nach proximal verschoben, so dass der eigentliche CRT-Stimulationsort nicht mehr optimal erreicht wird.

Die mittels üblicher Wahrnehmungseinheiten erfassten Zeitpunkte der linksventrikulären Wahrnehmung haben sich entsprechend verschoben, t1 bis t3 werden verspätet wahrgenommen. Da sich der vierte Elektrodenpol nun im Bereich des linken Vorhofes befindet, wird hier nun ein deutlich früheres Sense-Ereignis (t4) wahrgenommen.

Anhand der zeitlichen Verschiebung kann nun in einer Auswerteeinheit als Bestandteil einer Dislokations-Detektionseinheit einfach festgestellt werden, dass die CS-Elektrode um etwa einen Ringabstand nach proximal verschoben wurde.

Optional kann daraufhin die Stimulationssteuereinheit den Stimulationsort für die CRT-Stimulation korrigiert werden, z.B. eine Umschaltung von Pol 2 nach Pol 1.

Zu Verbesserung der Spezifität der Auswertung werden die entsprechenden Referenzzeiten bei unterschiedlichen Erregungsabläufen ausgewertet und so z.B. die Herzfrequenz und/oder die intraventrikulären Leitungszeiten im Merkmalsraum der Analyse erfasst. So könne reguläre IEGM-Exkursionen kompensiert werden, wie z.B. zirkadiane Schwankungen der Leitungszeiten oder Extrasystolen etc.

In der Figur 5 ist das Blockschaltbild einer Dislokations-Detektionseinheit 400 dargestellt. Die rechtsatriale (410), die vier CS-Elektrodenpole (420...450) und die rechtsventrikuläre (460) Elektrode sind jeweils mit einer Wahrnehmungseinheit (415, 425, ... 465) verbunden, um den Zeitpunkt der kardialen Erregung am Elektrodenpol erfassen zu können. Diese Zeitpunkte werden in einer Auswerteeinheit 470 erfasst und zunächst als Referenz in einer Matrix abgelegt. Diese Referenzmatrix wird für verschiedene Erregungszustände des Herzens abgelegt, so z.B. für verschiedene Herzfrequenzen etc. Anstelle dass die Dislokations-Detektionseinheit 400 eigene Wahrnehmungseinheiten (415, 425, ... 465) aufweist, kann sie auch auf die Wahrnehmungseinheiten 58, 62 und 66 zurückgreifen.

In der Figur 6 ist ein erweitertes Blockschaltbild dargestellt. Dieses entspricht dem aus Figur 5, erweitert um eine Umschaltung der CRT-Stimulationskonfiguration in Abhängigkeit der festgestellten Dislokationsausprägung. In dem in Figur 4 gezeigten Beispiel beträgt die Verschiebung der Elektrode etwa einen Ringabstand, so dass der Stimulationsort nun beispielsweise von dem Pol S2 nach S1 verschoben wird. So kann erreicht werden, dass die Stimulation trotz Dislokation immer noch am haemodynamisch optimalen Ort stattfindet. Die Umschaltung kann in der Stimulationssteuereinheit 54 implementiert sein und auf ein Ausgangssignal der Dislokations-Detektionseinheit 400 bzw. deren Auswerteeinheit 470 bzw. 570 ansprechen.

Ist die Dislokation so stark, dass der haemodynamisch günstige Stimulationsort nicht mehr erreicht werden kann, wird in Abhängigkeit der Programmierung die Stimulationskonfiguration des CRT-Systems auf einen sog. Backup-Mode umgeschaltet, um eine nicht notwendige und eventuell die Herzinsuffizient verschlechternde Stimulation zu vermeiden.

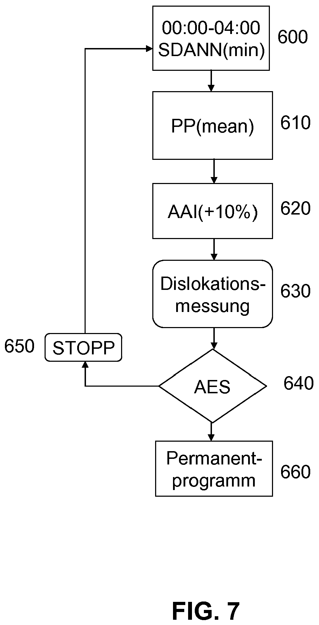

In der Figur 7 ist der Ablaufplan einer erfindungsgemäßen Implementierung der Dislokationsanalyse dargestellt. Zunächst wird geprüft, ob die definierte kardiale Konstellation angenommen werden kann (600). In diesem Beispiel wird die Analyse nur in der Nacht und zusätzlich nur dann gestartet, wenn sich der Parameter SDANN unterhalb einen aus dem Trendmittelwert bestimmten Grenzwertes befindet. Damit werden zirkadiane und andere die Messung beeinflussende Störgrößen der autonomen Regulation der Erregungsleitungszeiten minimiert.

Anschließend wird die mittlere Vorhoffrequenz (610) bestimmt und darauf basierend ein AAI-Mode mit einer Stimulationsfrequenz oberhalb der Vorhoffrequenz eingestellt (620). Bei dieser nun definierten Erregungsausbreitungssituation wird anschließend die eigentliche Dislokationsanalyse (630) ausgeführt. Diese Analyse läuft z.B. über 1-5 Minuten, um sehr spezifische Ergebnisse zu erhalten. Anschließend wird die Gültigkeit der Messung geprüft (640). Kriterium hierfür ist z.B. die Zahl der atrialen Extrasystolen bzw. inhibierten atrialen Stimulationen (640). Ist diese zu hoch, wird die Messung verworfen (650) und ggf. neu gestartet. Ist die Gültigkeit nachgewiesen, so schaltet der Stimulator zurück ins Permanentprogramm (660).

Die Erfindung bietet den Vorteil, dass eine CS-Elektrodendislokation frühzeitig erkannt und automatisch quantifiziert werden kann, so dass optional die CRT-Stimulationskonfiguration automatisch korrigiert werden kann.

## Patentansprüche

1. Herz-Therapie- und/oder Herz-Monitoring Gerät (100), welches mit wenigstens einer Elektrodenleitung (110, 112, 114) verbunden oder zu verbinden ist, die wenigstens einen ersten Wahrnehmungselektrodenpol (121) aufweist, und welches wenigstens einen zweiten Wahrnehmungselektrodenpol (122) aufweist oder mit einem solchen verbunden ist, wobei der erste und der zweite Wahrnehmungselektrodenpol (121, 122) im Betrieb des Herz-Therapie- und/oder Herz-Monitoring Gerätes (100) relativ zueinander beweglich sind,
wobei das Herz-Therapie- und/oder Herz-Monitoring Gerät (100) eine Dislokations-Detektionseinheit (400) aufweist, die sowohl mit dem ersten als auch mit dem zweiten Wahrnehmungselektrodenpol (121, 122) wenigstens mittelbar verbunden oder zu verbinden ist und die ausgebildet ist, zur Dislokations-Detektion einem jeweiligen kardialen Ereignis zuzuordnende Erfassungszeitpunkte an dem ersten Wahrnehmungselektrodenpol (121) und an dem zweiten Wahrnehmungselektrodenpol (122) relativ zueinander auszuwerten und ein Dislokations-Signal zu generieren, falls sich die relative zeitliche Beziehung von einem Typ kardialer Ereignisse zuzuordnenden Erfassungszeitpunkten über ein vorgegebenes oder jeweils bestimmtes Maß hinaus gegenüber einem zuvor erfassten Referenzwert ändert,
**dadurch gekennzeichnet dass** die Dislokations-Detektionseinheit (400) ausgebildet ist, ein Dislokationssignal zu liefern, das einen Grad einer zeitlichen Verschiebung von Erfassungszeitpunkten gegenüber dem zuvor erfassten Referenzwert widerspiegelt.

2. Herz-Therapie- und/oder Herz-Monitoring Gerät gemäß Anspruch 1, **dadurch gekennzeichnet, dass** die Dislokations-Detektionseinheit (400) ausgebildet ist, eine Zeitdifferenz zwischen zwei einem jeweiligen kardialen Ereignis zuzuordnenden Erfassungszeitpunkten als einem die relative zeitliche Beziehung beschreibenden Relativwert zu bestimmen und diesen Relativwert mit einem Referenzwert zu vergleichen, der aus einem oder mehreren Relativwerten eines oder mehrerer jeweils vorangegangener Herzzyklen bestimmt ist.

3. Herz-Therapie- und/oder Herz-Monitoring Gerät gemäß Anspruch 1 oder 2, **dadurch gekennzeichnet, dass** das Herz-Therapie- und/oder Herz-Monitoring Gerät (100) eine Stimulationseinheit (56, 64) aufweist, die mit einem Stimulationselektrodenpol verbunden oder zu verbinden ist, wobei die Dislokations-Detektionseinheit (400) dazu ausgebildet ist, die relative zeitliche Beziehung der Erfassungszeitpunkte eines jeweils stimulierten Ereignisses zu bestimmen und für eine Dislokations-Detektion heranzuziehen.

4. Herz-Therapie- und/oder Herz-Monitoring Gerät gemäß Anspruch 3, **dadurch gekennzeichnet, dass** die Stimulationseinheit (56, 60, 64) eine atriale Stimulationseinheit (60) ist und dass der Stimulationselektrodenpol ein atrialer Stimulationselektrodenpol zur Platzierung im Atrium eines Herzens ist.

5. Herz-Therapie- und/oder Herz-Monitoring Gerät gemäß einem der Ansprüche 1 bis 4, **dadurch gekennzeichnet, dass** die Elektrodenleitung mit dem ersten Wahrnehmungselektrodenpol eine Coronar-Sinus-Elektrodenleitung (114) ist.

6. Herz-Therapie- und/oder Herz-Monitoring Gerät gemäß einem der Ansprüche 1 bis 5, **dadurch gekennzeichnet, dass** der zweite Wahrnehmungselektrodenpol Teil einer rechtsventrikulären oder rechtsatrialen Elektrodenleitung (110, 112) ist.

7. Herz-Therapie- und/oder Herz-Monitoring Gerät gemäß einem der Ansprüche 1 bis 6, **dadurch gekennzeichnet, dass** zwei erste Wahrnehmungselektrodenpole vorgesehen sind und die Dislokations-Detektionseinheit (400) eine Auswerteeinheit (470) umfasst, die im Rahmen einer Zeitanalyse jeweilige Zeitdifferenzen zwischen jeweiligen Erfassungszeitpunkten an einem jeweiligen ersten Wahrnehmungselektrodenpol und dem zweiten Wahrnehmungselektrodenpol und ein Dislokationssignal bei hinreichender zeitlicher Verschiebung der Wahrnehmungszeitpunkte erzeugt, wobei die Dislokations-Detektionseinheit (400) einen jeweiligen Grad der zeitlichen Verschiebung durch Auswerten von mindestens zwei Zweitdifferenzen bestimmt, die sich zwischen je einem ersten Wahrnehmungspol und dem zweiten Wahrnehmungspol ergeben.

8. Herz-Therapie- und/oder Herz-Monitoring Gerät gemäß einem der Ansprüche 1 bis 7, **dadurch gekennzeichnet, dass** das Herz-Therapie- und/oder Herz-Monitoring Gerät (100) eine Stimulationssteuereinheit (54) umfasst, die mit wenigstens einer Stimulationseinheit (56, 60, 64) verbunden und ausgebildet ist, die Abgabe eines jeweiligen Stimulationsimpulses über wenigstens einen bestimmbaren Stimulationselektrodenpol in Abhängigkeit des Dislokationssignals zu steuern.

9. Herz-Therapie- und/oder Herz-Monitoring Gerät gemäß Anspruch 8, **dadurch gekennzeichnet, dass** die Stimulationssteuereinheit (54) dazu ausgebildet ist, einen von mehreren Stimulationselektrodenpolen in Abhängigkeit des Grades der zeitlichen Verschiebung zu bestimmen.

10. Herz-Therapie- und/oder Herz-Monitoring Gerät gemäß der Ansprüche 5 und 9, **dadurch gekennzeichnet, dass** die Stimulationssteuereinheit (54) dazu ausgebildet ist, eine automatische Umschaltung zwischen Stimulationselektrodenpolen an der Coronar-Sinus- Elektrodenleitung (114) in Abhängigkeit des Grades der Dislokation und/oder eine Abschaltung der Stimulation bei höhergradiger Dislokation zu bewirken.

11. Herz-Therapie- und/oder Herz-Monitoring Gerät gemäß einem der Ansprüche 1 bis 10, **dadurch gekennzeichnet, dass** die Dislokations-Detektionseinheit (400) dazu ausgebildet ist, eine Zeitanalyse zum Detektieren einer Dislokation bei einer durch das Herz-Therapie- und/oder Herz-Monitoring Gerät (100) erkannten oder eingestellten, definierten kardialen Konstellation durchzuführen.

12. Herz-Therapie- und/oder Herz-Monitoring Gerät gemäß einem der Ansprüche 1 bis 11, **dadurch gekennzeichnet, dass** die Dislokations-Detektionseinheit (400) dazu ausgebildet ist, eine Zeitanalyse zur Dislokations-Detektion durchzuführen, die eine Zeit-Korrektur basierend auf einer interventrikulären Signallaufzeit beinhaltet.

13. Herz-Therapie- und/oder Herz-Monitoring Gerät gemäß der Ansprüche 5 und 12, **dadurch gekennzeichnet, dass** das Herz-Therapie- und/oder Herz-Monitoring Gerät (100) dazu ausgebildet ist, die interventrikuläre Signallaufzeit an mindestens zwei wahrnehmungselektrodenpolen an der Coronar-Sinus- Elektrodenleitung (114) zu messen.

14. Herz-Therapie- und/oder Herz-Monitoring Gerät gemäß einem der Ansprüche 1 bis 11, **dadurch gekennzeichnet, dass** das Herz-Therapie- und/oder Herz-Monitoring Gerät (100) ein implantierbarer biventrikulärer Herzschrittmacher und/oder Kardioverter/Defibrillator ist.

## Claims

1. A cardiac therapy device and/or a cardiac monitoring device (100), which device is connected or is to be connected to at least one electrode lead (110, 112, 114), which has at least one first sensing electrode pole (121), and which device has at least one second sensing electrode pole (122) or is connected to such a sensing electrode pole, wherein the first and the second sensing electrode pole (121, 122) are movable relative to one another during operation of the cardiac therapy device and/or the cardiac monitoring device (100),
wherein the cardiac therapy device and/or cardiac monitoring device (100) has a dislocation detection unit (400), which is connected or is to be connected at least indirectly to the first and to the second sensing electrode pole (121, 122) and which is designed, for dislocation detection, to evaluate detection times, which are to be associated with a particular cardiac event, at the first sensing electrode pole (121) and at the second sensing electrode pole (122) relative to one another and to generate a dislocation signal if the relative temporal relationship of detection times to be associated with a particular type of cardiac event to a previously detected reference value changes beyond a predefined or determined extent,
**characterised in that** the dislocation detection unit (400) is designed to deliver a dislocation signal which reflects a degree of a temporal shift of detection times compared to the previously detected reference value.

2. The cardiac therapy device and/or the cardiac monitoring device according to claim 1, **characterised in that** the dislocation detection unit (400) is designed to determine a time difference between two detection times to be associated with a particular cardiac event as a relative value describing the relative temporal relationship and to compare this relative value with a reference value, which is determined from one or more relative values of one or more previous cardiac cycles.

3. The cardiac therapy device and/or the cardiac monitoring device according to claim 1 or 2, **characterised in that** the cardiac therapy device and/or cardiac monitoring device (100) has a stimulation unit (56, 64), which is connected or is to be connected to a stimulation electrode pole, wherein the dislocation detection unit (400) is designed to determine the relative temporal relationship of the detection times of a particular stimulated event and to use this for dislocation detection.

4. The cardiac therapy device and/or the cardiac monitoring device according to claim 3, **characterised in that** the stimulation unit (56, 60, 64) is an atrial stimulation unit (60), and **in that** the stimulation electrode pole is an atrial stimulation electrode pole for placement in the atrium of a heart.

5. The cardiac therapy device and/or the cardiac monitoring device according to any one of claims 1 to 4, **characterised in that** the electrode lead with the first sensing electrode pole is a coronary sinus electrode lead (114).

6. The cardiac therapy device and/or the cardiac monitoring device according to any one of claims 1 to 5, **characterised in that** the second sensing electrode pole is part of a right-ventricular or right-atrial electrode lead (110, 112).

7. The cardiac therapy device and/or the cardiac monitoring device according to any one of claims 1 to 6, **characterised in that** two first sensing electrode poles are provided and the dislocation detection unit (400) comprises an evaluation unit (470), which, within the scope of a time analysis, generates time differences between detection times at a particular first sensing electrode pole and the second sensing electrode pole and generates a dislocation signal given an adequate temporal shift of the sensing times, wherein the dislocation detection unit (400) determines the extent of the temporal shift by evaluating at least two time differences established between each first sensing pole and the second sensing pole.

8. The cardiac therapy device and/or the cardiac monitoring device according to any one of claims 1 to 7, **characterised in that** the cardiac therapy device and/or cardiac monitoring device (100) comprises a stimulation control unit (54), which is connected to at least one stimulation unit (56, 60, 64) and which is designed to control the delivery of a stimulation pulse via at least one determinable stimulation electrode pole depending on the dislocation signal.

9. The cardiac therapy device and/or the cardiac monitoring device according to claim 8, **characterised in that** the stimulation control unit (54) is designed to determine one of a number of stimulation electrode poles depending on the extent of the temporal shift.

10. The cardiac therapy device and/or the cardiac monitoring device according to claims 5 and 9, **characterised in that** the stimulation control unit (54) is designed to bring about an automatic switchover between stimulation electrode poles at the coronary sinus electrode lead (114) depending on the extent of the dislocation and/or switch-off of the stimulation if the dislocation is extreme.

11. The cardiac therapy device and/or the cardiac monitoring device according to any one of claims 1 to 10, **characterised in that** the dislocation detection unit (400) is designed to perform a time analysis for detection of a dislocation in the event of a defined cardiac constellation identified or set by the cardiac therapy device and/or the cardiac monitoring device (100).

12. The cardiac therapy device and/or the cardiac monitoring device according to any one of claims 1 to 11, **characterised in that** the dislocation detection unit (400) is designed to perform a time analysis for dislocation detection, which time analysis includes a time correction based on an interventricular signal propagation time.

13. The cardiac therapy device and/or the cardiac monitoring device according to any one of claims 5 and 12, **characterised in that** the cardiac therapy device and/or cardiac monitoring device (100) is designed to measure the interventricular signal propagation time at least at two sensing electrode poles of the coronary sinus electrode lead (114).

14. The cardiac therapy device and/or the cardiac monitoring device according to any one of claims 1 to 11, **characterised in that** the cardiac therapy device and/or the cardiac monitoring device (100) is an implantable biventricular cardiac pacemaker and/or cardioverter/defibrillator.

## Revendications

1. Appareil de thérapie cardiaque et/ou de surveillance cardiaque (100), lequel est relié, ou est à relier, avec au moins une ligne d'électrode (110, 112, 114), qui présente au moins un premier pôle d'électrode de détection (121), et lequel présente au moins un deuxième pôle d'électrode de détection (122) ou est relié avec un tel pôle, où les premier et deuxième pôles d'électrode de détection (121, 122) sont mobiles l'un par rapport à l'autre pendant le fonctionnement de l'appareil de thérapie cardiaque et/ou de surveillance cardiaque (100),
où l'appareil de thérapie cardiaque et/ou de surveillance cardiaque (100) présente une unité de détection de délocalisation (400), qui est reliée, ou est à relier, au moins indirectement, à la fois avec le premier pôle d'électrode de détection et le deuxième (121, 122) et qui est conçue pour évaluer l'un par rapport à l'autre des moments de détection à associer à un évènement cardiaque respectivement sur le premier pôle d'électrode de détection (121) et sur le deuxième pôle de détection (122) pour la détection de la délocalisation et pour générer un signal de délocalisation dans le cas où la relation relative avec le temps des moments de détection à associer à un type d'évènement cardiaque se modifie au-delà d'une valeur prédéfinie ou respectivement déterminée par rapport à une valeur de référence déterminée auparavant,
**caractérisé en ce que** l'unité de détection de délocalisation (400) est conçue pour délivrer un signal de délocalisation qui représente un niveau d'un décalage dans le temps de moments de détection par rapport à la valeur de référence déterminée auparavant.

2. Appareil de thérapie cardiaque et/ou de surveillance cardiaque selon la revendication 1, **caractérisé en ce que** l'unité de détection de délocalisation (400) est conçue pour déterminer une différence de temps entre deux moments de détection à associer à un évènement cardiaque respectif sous la forme d'une valeur relative décrivant la relation relative avec le temps et pour comparer cette valeur relative avec une valeur de référence qui est déterminée à partir d'une ou de plusieurs valeurs relatives d'un ou de plusieurs cycles cardiaques respectivement antérieurs.

3. Appareil de thérapie cardiaque et/ou de surveillance cardiaque selon la revendication 1 ou 2, **caractérisé en ce que** l'appareil de thérapie cardiaque et/ou de surveillance cardiaque (100) présente une unité de stimulation (56, 64), qui est reliée, ou est à relier, avec un pôle d'électrode de stimulation, où l'unité de détection de délocalisation (400) est conçue pour déterminer la relation relative avec le temps du moment de détection d'un évènement respectivement stimulé et pour faire appel à une détection de délocalisation.

4. Appareil de thérapie cardiaque et/ou de surveillance cardiaque selon la revendication 3, **caractérisé en ce que** l'unité de stimulation (56, 60, 64) est une unité de stimulation atriale (60) et que le pôle d'électrode de stimulation est un pôle d'électrode de stimulation atrial pour la mise en place dans l'oreillette du coeur.

5. Appareil de thérapie cardiaque et/ou de surveillance cardiaque selon l'une des revendications 1 à 4, **caractérisé en ce que** la ligne d'électrode avec le premier pôle d'électrode de détection est une ligne d'électrode du sinus coronaire (114).

6. Appareil de thérapie cardiaque et/ou de surveillance cardiaque selon l'une des revendications 1 à 5, **caractérisé en ce que** le deuxième pôle d'électrode de détection est une partie d'une ligne d'électrode ventriculaire droite ou atriale droite (110, 112).

7. Appareil de thérapie cardiaque et/ou de surveillance cardiaque selon l'une des revendications 1 à 6, **caractérisé en ce que** deux premiers pôles d'électrode de détection sont prévus et l'unité de détection de délocalisation (400) comprend une unité d'exploitation (470), qui, dans le cadre d'une analyse dans le temps génère des différences de temps respectives entre des moments de détection au niveau respectivement d'un premier pôle d'électrode de détection respectif et du deuxième pôle d'électrode de détection et génère un signal de délocalisation lors d'un décalage de temps suffisant des moments de détection, où l'unité de détection de délocalisation (400) détermine une ampleur respective de décalage de temps par une évaluation d'au moins deux différences de temps qui se produisent entre respectivement un premier pôle de détection et le deuxième pôle de détection.

8. Appareil de thérapie cardiaque et/ou de surveillance cardiaque selon l'une des revendications 1 à 7, **caractérisé en ce que** l'appareil de thérapie cardiaque et/ou de surveillance cardiaque (100) comprend une unité de commande de stimulation (54), qui est reliée avec au moins une unité de stimulation (56, 60, 64) et est conçue pour commander l'émission d'une impulsion de stimulation respective par l'intermédiaire d'au moins un pôle d'électrode de stimulation pouvant être défini en fonction du signal de délocalisation.

9. Appareil de thérapie cardiaque et/ou de surveillance cardiaque selon la revendication 8, **caractérisé en ce que** l'unité de commande de stimulation (54) est conçue pour définir un parmi plusieurs pôles d'électrode de stimulation en fonction de l'ampleur du décalage de temps.

10. Appareil de thérapie cardiaque et/ou de surveillance cardiaque selon les revendications 5 et 9, **caractérisé en ce que** l'unité de commande de stimulation (54) est conçue pour provoquer une commutation automatique entre des pôles d'électrode de stimulation sur la ligne d'électrode du sinus coronaire (114) en fonction de l'ampleur de la délocalisation et/ou un arrêt de la stimulation pour une délocalisation d'ampleur élevée.

11. Appareil de thérapie cardiaque et/ou de surveillance cardiaque selon l'une des revendications 1 à 10, **caractérisé en ce que** l'unité de détection de délocalisation (400) est conçue pour effectuer une analyse dans le temps pour la détection d'une délocalisation lors d'une configuration cardiaque définie, détectée ou réglée par l'appareil de thérapie cardiaque et/ou de surveillance cardiaque (100).

12. Appareil de thérapie cardiaque et/ou de surveillance cardiaque selon l'une des revendications 1 à 11, **caractérisé en ce que** l'unité de détection de délocalisation (400) est conçue pour exécuter une analyse dans le temps pour la détection de délocalisations qui contient une correction du temps se basant sur une durée de signal inter-ventriculaire.

13. Appareil de thérapie cardiaque et/ou de surveillance cardiaque selon les revendications 5 et 12, **caractérisé en ce que** l'appareil de thérapie cardiaque et/ou de surveillance cardiaque (100) est conçu pour mesurer la durée de signal inter-ventriculaire à au moins deux pôles d'électrode de détection sur la ligne d'électrode de sinus coronaire (114).

14. Appareil de thérapie cardiaque et/ou de surveillance cardiaque selon l'une des revendications 1 à 11, **caractérisé en ce que** l'appareil de thérapie cardiaque et/ou de surveillance cardiaque (100) est un stimulateur cardiaque biventriculaire implantable et/ou un cardioverteur/défibrillateur.
